(19) Europäisches Patentamt
European Patent Office
Office européen des brevets

(11) Veröffentlichungsnummer: **0 080 106**
**B1**

# (12) EUROPÄISCHE PATENTSCHRIFT

(45) Veröffentlichungstag der Patentschrift: **16.10.85**

(51) Int. Cl.⁴: **C 07 C 125/06**

(21) Anmeldenummer: **82110322.3**

(22) Anmeldetag: **09.11.82**

## (54) Verfahren zur Herstellung von N-substituierten N-Isocyanatocarbonyl-carbamaten.

(30) Priorität: **21.11.81 DE 3146230**

(43) Veröffentlichungstag der Anmeldung: **01.06.83 Patentblatt 83/22**

(45) Bekanntmachung des Hinweises auf die Patenterteilung: **16.10.85 Patentblatt 85/42**

(84) Benannte Vertragsstaaten: **AT BE CH DE FR GB IT LI NL**

(56) Entgegenhaltungen: **SYNTHESIS, 1980, Seite 112, Georg Thieme Verlag, Stuttgart, DE. V.I. GORBATENKO et al.: "Reactions of chlorocarbonyl isocyanate with carbamates and ureas containing an N-H bond"**

(73) Patentinhaber: **BAYER AG, Konzernverwaltung RP Patentabteilung, D-5090 Leverkusen 1 Bayerwerk (DE)**

(72) Erfinder: **Kühle, Engelbert, Dr., Von-Bodelschwingh-Strasse 42, D-5060 Bergisch-Gladbach 2 (DE)**
Erfinder: **Hagemann, Hermann, Dr., Kandinsky-Strasse 52, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein Verfahren zur Herstellung von grösstenteils neuen N-substituierten N-Isocyanatocarbonyl-carbamaten, die zum Beispiel als Zwischenprodukte für die Herstellung von Schädlingsbekämpfungsmitteln Verwendung finden können.

Es ist bereits bekannt, dass man N-substituierte N-Isocyanatocarbonyl-O-alkyl-carbamate erhält, wenn man entsprechende N-substituierte Carbamidsäure-O-alkylester mit Chlorcarbonylisocyanat in Gegenwart eines tertiären Amins als Säurebinder gegebenenfalls in Gegenwart eines Lösungsmittels umsetzt (Synthesis 1980, 112).

Dieses Verfahren hat jedoch den Nachteil, dass bei der Umsetzung als Nebenprodukt ein Salz entsteht, das die Wirtschaftlichkeit des Verfahrens beeinträchtigt.

Gegenstand der vorliegenden Erfindung ist ein Verfahren zur Herstellung von N-substituierten N-Isocyanatocarbonyl-carbamaten der Formel I,

R-N-COOR$^1$<br>
|<br>
CO-NCO (I)

in welcher

R und R$^1$ gleich oder verschieden sind und einzeln für gegebenenfalls substituierte Reste aus der aliphatischen, cycloaliphatischen, araliphatischen oder aromatischen Reihe stehen,

das dadurch gekennzeichnet ist, dass man N-substituierte Carbamidsäureester der Formel II

R-NH-COOR$^1$ (II)

in der

R und R$^1$ die oben angegebene Bedeutung haben, mit Chlorcarbonylisocyanat der Formel III

Cl-CO-NCO (III)

in einem Molverhältnis von Chlorcarbonylisocyanat (III) zu Carbamidsäureester (II) von 1:1 bis 10:1 bei Temperaturen von 50 bis 200°C in einem Verdünnungsmittel ohne Zusatz von Säurebinder umsetzt.

Es wurden ferner die neuen N-substituierten N-Isocyanatocarbonyl-carbamate der Formel Ia

R-N-COOR$^2$<br>
|<br>
CO-NCO (Ia)

gefunden, in welcher

R für gegebenenfalls substituierte Reste aus der aliphatischen, cycloaliphatischen, araliphatischen oder aromatischen Reihe steht und

R$^2$ für gegebenenfalls substituierte Reste aus der cycloaliphatischen, araliphatischen oder aromatischen Reihe steht.

Die neuen N-substituierten N-Isocyanatocarbonyl-carbamate der Formel Ia können dadurch erhalten werden, dass man N-substituierte Carbamidsäureester der Formel IIa

R-NH-COOR$^2$ (IIa)

in der

R und R$^2$ die oben angegebene Bedeutung haben, mit Chlorcarbonylisocyanat der Formel III

Cl-CO-NCO (III)

in einem Molverhältnis von Chlorcarbonylisocyanat (III) zu Carbamidsäureester (II) von 1:1 bis 10:1 bei Temperaturen von 50 bis 200°C in einem Verdünnungsmittel ohne Zusatz von Säurebinder umsetzt.

Es ist als ausgesprochen überraschend zu bezeichnen, dass die erfindungsgemässe Umsetzung bei erhöhter Temperatur so glatt und ohne Bildung von Nebenprodukten abläuft. Es war vielmehr zu erwarten, dass die als Ausgangsmaterialien einzusetzenden N-substituierten Carbamidsäureester der Formel II bzw. IIa vor der Reaktion mit dem Chlorcarbonylisocyanat der Formel III bei der erhöhten Temperatur rückgespalten werden in Isocyanate und Alkohole bzw. Phenole, die dann ihrerseits mit dem Chlorcarbonylisocyanat der Formel III reagieren könnten.

Weiterhin ist hervorzuheben, dass das erfindungsgemässe Verfahren gegenüber dem bekannten Verfahren den Vorteil bietet, dass als Nebenprodukt gasförmiger Chlorwasserstoff entsteht, der kontinuierlich aus dem Reaktionssystem entweicht bzw. abgeführt werden kann. Somit entfällt die Abtrennung und Aufarbeitung des beim bekannten Verfahren anfallenden Salzes, was zu Schwierigkeiten hätte führen können im Hinblick auf die sehr hydrolyseempfindlichen Reaktionsprodukte. Die N-substituierten N-Isocyanatocarbonyl-carbamate werden nach dem erfindungsgemässen Verfahren in sehr guten Ausbeuten und hoher Reinheit erhalten.

Verwendet man beispielsweise N-Methylcarbamidsäure-O-phenylester und Chlorcarbonylisocyanat als Ausgangsstoffe, so kann der Reaktionsverlauf durch das folgende Formelschema wiedergegeben werden:

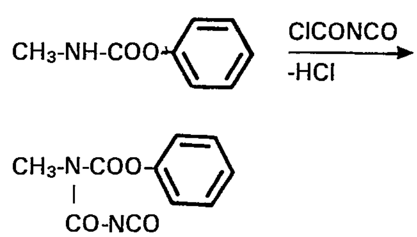


Die beim erfindungsgemässen Verfahren als Ausgangsmaterialien zu verwendenden N-substituierten Carbamidsäureester sind durch die allgemeine Formel II bzw. IIa definiert.

In diesen Formeln steht als gegebenenfalls substituierter aliphatischer Rest R und R$^1$ gegebenenfalls substituiertes Alkyl, Alkenyl oder Alkinyl.

Als gegebenenfalls substituierter cycloaliphatischer Rest R, R$^1$ und R$^2$ steht ein gegebenenfalls substituierter Cycloalkylrest oder ein gegebenenfalls substituierter Cycloalkenylrest.

Als gegebenenfalls substituierter araliphatischer Rest R, R$^1$ und R$^2$ steht ein gegebenenfalls substitu-

ierter Aralkylrest oder ein gegebenenfalls substituierter Aralkenylrest.

Als gegebenenfalls substituierter aromatischer Rest R, $R^1$ und $R^2$ steht ein gegebenenfalls substituierter Arylrest.

Als gegebenenfalls substituiertes Alkyl R und $R^1$ steht geradkettiges oder verzweigtes Alkyl mit 1 bis 20, vorzugsweise 1 bis 10, insbesondere 1 bis 5 Kohlenstoffatomen. Beispielhaft seien gegebenenfalls substituiertes Methyl, Ethyl, n- und i-Propyl, n-, i-, sec.- und tert.-Butyl genannt.

Als gegebenenfalls substituiertes Alkenyl R und $R^1$ steht geradkettiges oder verzweigtes Alkenyl mit 2 bis 10, vorzugsweise 2 bis 8, insbesondere 2 bis 4 Kohlenstoffatomen. Beispielhaft seien gegebenenfalls substituiertes Ethenyl, Propenyl-(1), Propenyl-(2) und Butenyl genannt.

Als gegebenenfalls substituiertes Alkinyl R und $R^1$ steht geradkettiges oder verzweigtes Alkinyl mit 2 bis 10, vorzugsweise 2 bis 8, insbesondere 2 bis 4 Kohlenstoffatomen.

Beispielhaft seien gegebenenfalls substituiertes Ethinyl, Propinyl-(1), Propinyl-(3) und Butinyl genannt.

Als gegebenenfalls substituierter Cycloalkylrest R, $R^1$ und $R^2$ steht ein mono- und bicyclisches Cycloalkyl mit 3 bis 8, vorzugsweise 5 bis 8, insbesondere 5 oder 6 Kohlenstoffatomen. Beispielhaft seien gegebenenfalls substituiertes Cyclopentyl oder Cyclohexyl genannt.

Als gegebenenfalls substituierter Cycloaklenylrest R, $R^1$ und $R^2$ steht ein monocyclisches Cycloalkenyl mit 5 oder 6 Kohlenstoffatomen und 1 oder 2 Doppelbindungen.

Als gegebenenfalls substituierter Aralkylrest R, $R^1$ und $R^2$ steht gegebenenfalls im Arylteil und/oder Alkylteil substituiertes Aralkyl mit 6 bis 10, vorzugsweise 6 Kohlenstoffatomen im Arylteil und 1 bis 6, vorzugsweise 1 bis 4 Kohlenstoffatomen, speziell 1 oder 2 Kohlenstoffatomen im Alkylteil, wobei der Alkylteil geradkettig oder verzweigt sein kann. Beispielhaft seien gegebenenfalls substituiertes Benzyl und Phenylethyl genannt.

Gegebenenfalls substituiertes Aralkenyl R, $R^1$ und $R^2$ entspricht in seinem Arylteil dem Aralkylrest in R, $R^1$ und $R^2$. Es enthält im Alkenylteil vorzugsweise 2 bis 6, insbesondere 2 oder 3 Kohlenstoffatome, vorzugsweise 1 Doppelbindung.

Als gegebenenfalls substituiertes Aryl R, $R^1$ und $R^2$ steht Aryl mit 6 bis 10 Kohlenstoffatomen im Arylteil. Beispielhaft seien gegebenenfalls substituiertes Phenyl oder Naphthyl, insbesondere Phenyl, genannt.

Die in den Definitionen von R, $R^1$ und $R^2$ genannten substituierten Reste können einen oder mehrere, gleiche oder verschiedene Substituenten tragen.

Als Substituenten für die in R und $R^1$ definierten substituierten Alkylreste, substituierten Alkenylreste und substituierten Alkinylreste seien beispielhaft aufgeführt: Alkoxy mit vorzugsweise 1 bis 4, insbesondere 1 bis 2 Kohlenstoffatomen, wie Methoxy, Ethoxy, n- oder i-Propyloxy und n-, i-, sec.- und tert.-Butyloxy, Alkylthio mit vorzugsweise 1 bis 4, insbesondere 1 oder 2 Kohlenstoffatomen, wie Methylthio, Ethylthio, n- und iso-Propylthio und n-, i-, sec.- und tert.-Butylthio; Halogen, vorzugsweise Fluor, Chlor, Brom und Jod, insbesondere Chlor und Brom, Cyano und Nitro.

Als Substituenten für das in R, $R^1$ und $R^2$ definierte substituierte Cycloalkyl oder substituierte Cycloalkenyl seien beispielhaft aufgeführt: Alkyl mit 1 bis 4, insbesondere 1 oder 2 Kohlenstoffatomen, wie Methyl, Ethyl, n- und i-Propyl, n-, i-, sec.- und tert.-Butyl.

Als Substituenten des Arylteils für das in R, $R^1$ und $R^2$ definierte substituierte Aralkyl seien beispielhaft aufgeführt: Alkyl mit 1 bis 4, insbesondere 1 oder 3 Kohlenstoffatomen: Alkoxy mit 1 bis 4, insbesondere 1 oder 2 Kohlenstoffatomen; Halogen, vorzugsweise Fluor, Chlor, Brom und Jod, insbesondere Fluor oder Chlor; Nitro; Cyan und Trifluormethyl.

Als Substituenten für den in R, $R^1$ und $R^2$ definierten Arylrest seien beispielhaft genannt: Alkyl mit 1 bis 4, insbesondere 1 oder 2 Kohlenstoffatomen: Alkoxy mit 1 bis 4, insbesondere 1 oder 2 Kohlenstoffatomen, Halogen, vorzugsweise Fluor, Chlor, Brom und Jod, insbesondere Fluor oder Chlor; Cyano; Nitro; Trifluormethyl oder ein ankondensierter heterocyclischer Ring.

Die Erfindung betrifft ferner insbesondere neue Verbindungen der Formel Ia, in welcher

R und $R^2$ für einen gegebenenfalls durch Methyl oder Ethyl substituierten Cyclohexylrest, gegebenenfalls durch Fluor, Chlor, Nitro, Methyl, Ethyl, Methoxy, Ethoxy, n- und i-Propoxy, Nitro, Cyano, Trifluormethyl oder Dimethyldihydrofuranyl substituiertes Phenyl, gegebenenfalls durch Fluor, Chlor, Methyl, Ethyl, Methoxy, Ethoxy, n- und i-Propoxy, Nitro, Trifluormethyl substituiertes Naphthyl, für gegebenenfalls im Phenylteil durch Methyl, Ethyl, Methoxy, Ethoxy, Fluor, Chlor, Nitro, Cyan und Trifluormethyl substituiertes Phenylmethyl oder Phenylethyl steht und

R ausserdem für einen gegebenenfalls durch Fluor, Chlor, Methoxy, Ethoxy, Methylthio, Ethylthio, Cyano oder Nitro substituierten Alkylrest mit 1 bis 6 Kohlenstoffatomen oder für $C_2$-$C_4$-Alkenyl steht.

Die Ausgangsverbindungen der Formel II bzw. IIa sind bekannt und können nach bekannten Verfahren z.B. durch Addition von Alkoholen oder Phenolen an Isocyanate [vgl. Houben-Weyl: Methoden der Organ. Chemie, 4. Aufl., Bd. 8, S. 141 (1952)] oder durch Umsetzung von Kohlensäureesterchloriden mit primären Aminen (vgl. loc. cit. S. 138) hergestellt werden.

Das weiter als Ausgangsstoff zu verwendende Chlorcarbonylisocyanat ist bekannt [Angew. Chemie 89, 789 (1977)].

Das erfindungsgemässe Verfahren wird in Anwesenheit eines Verdünnungsmittels durchgeführt. Als Verdünnungsmittel kommen alle inerten Lösungsmittel in Frage wie Kohlenwasserstoffe, z.B. Toluol oder Xylol, chlorierte Kohlenwasserstoffe, z.B. Chlorbenzol oder Ether, z.B. Dioxan.

Das erfindungsgemässe Verfahren wird ohne Zusatz eines säurebindenden Mittels durchgeführt. Zweckmässigerweise wird das Chlorcarbonylisocyanat in Lösung vorgelelgt und soll im Überschuss im Reaktionsmedium vorhanden sein. Das Molverhältnis Chlorcarbonylisocyanat zu Carbamidsäureester kann in einem Bereich von 1:1 bis 1:10 variieren.

Die Reaktionstemperaturen können in einem Bereich von 50-200°C variiert werden.

Das erfindungsgemässe Verfahren wird vorzugsweise bei Normaldruck durchgeführt.

In einer bevorzugten Ausführungsform des erfindungsgemässen Verfahrens wird der N-substituierte Carbamidsäureester der Formel II bzw. IIa in einem der angegebenen Verdünnungsmittel gelöst und zu dem Chlorcarbonylisocyanat der Formel III, das in einem der angegebenen Verdünnungsmittel gelöst ist, zugetropft. Die Reaktionslösung wird erhitzt, wobei ungefähr ab 95°C Chlorwasserstoff als Gas entweicht. Nach mehrstündigem Erhitzen wird das Reaktionsgemisch durch Abdestillieren des Lösungsmittels aufgearbeitet. Die Isolierung der Verbindungen erfolgt in einfacher Weise durch Destillation.

Bei längerem Stehen dimerisieren die erfindungsgemässen Verbindungen sehr leicht, was durch Kristallisieren oder Zähflüssigkeit festgestellt werden kann. Durch thermische Behandlung z.B. durch Redestillation spalten die Dimeren leicht in die monomeren Verbindungen zurück.

Die erfindungsgemässen neuen Verbindungen der Formel Ia können als Zwischenprodukte zur Herstellung neuer N-sulfenierter Biuret-N''-carbonsäureester verwendet werden. Man setzt die neuen N-substituierten N-Isocyanato-carbonyl-carbamate der Formel Ia mit einem Sulfenamid der Formel

$$\begin{array}{l} HN\text{-}SR^4 \\ | \\ R^3 \end{array}$$

in welcher

$R^3$ für Wasserstoff, einen gegebenenfalls substituierten aliphatischen, cycloaliphatischen, araliphatischen oder aromatischen Rest steht und

$R^4$ für einen Trihalogenmethylrest steht,

in Gegenwart eines Verdünnungsmittels bei Temperaturen zwischen 0 und 150°C, vorzugsweise bei 20 bis 120°C um. Die Aufarbeitung des Reaktionsgemisches erfolgt durch Abdestillieren des Verdünnungsmittels. Die Endprodukte fallen entweder ölig oder in kristalliner Form an. Diese Verbindungen können als Pflanzenschutzmittel verwendet werden. Sie besitzen u.a. eine sehr gute fungizide Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trockenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 bis 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

Das erfindungsgemässe Verfahren sei anhand der folgenden Herstellungsbeispiele erläutert:

*Beispiel 1*

$(CH_3)_3C-CH_2-N-COO-$ 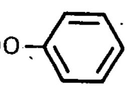

CONCO

62 g (0,3 Mol) N-Neopentylcarbamidsäure-O-phenylester werden in 200 ml trockenem Chlorbenzol gelöst und zu einer Lösung von 35 g (0,33 Mol) Chlorcarbonylisocyanat in 70 ml trockenem Chlorbenzol getropft. Bei Raumtemperatur findet keine merkliche Reaktion statt. Beim Erhitzen der Reaktionslösung entwickelt sich ab etwa 95° fortlaufend Chlorwasserstoff. Nach etwa 1,5 - 2 Std. ist diese Gasentwicklung mit Erreichen der Siedetemperatur des Chlorbenzols beendet. Man destilliert das Lösungsmittel im Vakuum ab. Der Rückstand liefert durch Hochvakuumdestillation 68 g N-Neopentyl-N--isocyanato-carbonyl-carbamidsäure-O-phenylester vom Siedepunkt 111 bis 112°C/0,1 Torr (88% d.Th.).

In analoger Weise erhält man folgende Verbindungen der Formel I

$$\begin{array}{c} R-N-COOR^1 \\ | \\ CO-NCO \end{array} \qquad (I)$$

| Beispiel Nr. | R | $R^1$ | Siedepunkt °C/Torr |
|---|---|---|---|
| 2 | $CH_3-$ | $C_6H_5-$ | 97/0,08 |
| 3 | $i-C_3H_7-$ | $C_6H_5-$ | 125/0,1 |
| 4 | $i-C_4H_9-$ | $C_6H_5-$ | 95-96/0,09 |
| 5 | 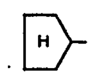 | $C_6H_5-$ | 128-130/0,08 |
| 6 | 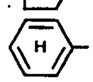 | $C_6H_5-$ | 145-150/0,1 |
| 7 | 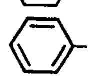 | $C_6H_5-$ | 162/0,1 |
| 8 | $CH_3-$ | 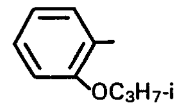 | 132-133/0,1 |
| 9 | $CH_3-$ | 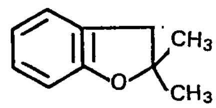 | 152/0,1 |
| 10 | 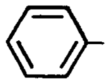 | $C_2H_5$ | 130-135/0,8 |
| 11 | $(CH_3)_3C-CH_2-$ | $C_2H_5$ | 70-74/0,2 |

*Herstellung eines N-sulfenylierten Biuret-N''-carbonsäure-esters*

*Beispiel I*

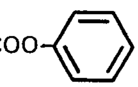

$$\begin{array}{l} CH_3-N\langle \begin{array}{l} COO- \\ CONHCON-CH_3 \\ \qquad\quad | \\ \qquad\quad SCFCl_2 \end{array} \end{array}$$

14 g (0,063 Mol) N-Methyl-N-(isocyanatocarbonyl)-O-phenyl-carbamat werden in 50 ml Dioxan gelöst und bei Raumtemperatur tropfenweise mit 10,5 g (0,07 Mol) Fluordichlormethansulfenyl-N--methylamid versetzt. Hierbei steigt die Temperatur bis 60°C an. Man engt die Reaktionslösung im Vakuum ein und kristallisiert den Rückstand aus Methanol um. Fp: 119 - 120°C, Ausbeute 18 g (78% d.Th.).

*Beispiel*

Pyricularia-Test (Reis)/protektiv

Lösungsmittel: 12,5 Gew.-Teile Aceton
Emulgator: 0,3 Gew.-Teile Alkylarylpolyglykolether

Zur Herstellung einer zweckmässigen Wirkstoffzubereitung vermischt man 1 Gew.-Teil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Trofnässe. Nach dem Abtrocknen des Spritzbelages werden die Pflanzen mit einer wässrigen Sporensuspension von Pyricularia oryzae inokuliert. Anschliessend werden die Pflanzen in einem Gewächshaus bei 100% rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigt bei diesem Test die Verbindung gemäss Herstellungsbeispiel I.

**Patentansprüche**

1. Verfahren zur Herstellung von N-substituierten N-Isocyanatocarbnyl-carbamaten der Formel I

$$\begin{array}{l} R\text{-}N\text{-}COOR^1 \\ \quad | \\ \quad CO\text{-}NCO \end{array} \qquad (I)$$

in welcher

R und $R^1$ gleich oder verschieden sind und einzeln für gegebenenfalls substituierte Reste aus der aliphatischen, cycloaliphatischen, araliphatischen oder aromatischen Reihe stehen,

dadurch gekennzeichnet, dass man N-substituierte Carbamidsäureester der Formel II

$$R\text{-}NH\text{-}COOR^1 \qquad (II)$$

in der

R und $R^1$ die oben angegebene Bedeutung haben, mit Chlorcarbonylisocyanat der Formel III

$$Cl\text{-}CO\text{-}NCO \qquad (III)$$

in einem Molverhältnis von Chlorcarbonylisocyanat (III) zu Carbamidsäureester (II) von 1:1 bis 10:1 bei Temperaturen von 50 bis 200°C in einem Verdünnungsmittel ohne Zusatz von Säurebinder umsetzt.

2. N-substituierte N-Isocyanatocarbonylcarbamate der Formel Ia

$$\begin{array}{l} R\text{-}N\text{-}COOR^2 \\ \quad | \\ \quad CO\text{-}NCO \end{array} \qquad (Ia)$$

in welcher

R für gegebenenfalls substituierte Reste aus der aliphatischen, cycloaliphatischen, araliphatischen oder aromatischen Reihe steht, und

$R^2$ für gegebenenfalls substituierte Reste aus der cycloaliphatischen, araliphatischen oder aromatischen Reihe steht.

3. N-substituierte N-Isocyanatocarbonylcarbamate gemäss Anspruch 2, wobei in der Formel Ia

R und $R^2$ für einen gegebenenfalls durch Methyl oder Ethyl substituierten Cyclohexylrest, gegebenenfalls durch Fluor, Chlor, Methyl, Ethyl, Methoxy, Ethoxy, n- und i-Propoxy, Nitro, Cyano, Trifluormethyl oder Dimethyldihydrofuranyl substituiertes Phenyl, gegebenenfalls durch Fluor, Chlor, Methyl, Ethyl, Methoxy, Ethoxy, n- und i-Propoxy, Nitro, Trifluormethyl substituiertes Naphthyl, für gegebenenfalls im Phenylteil durch Methyl, Ethyl, Methoxy, Ethoxy, Fluor, Chlor, Nitro, Cyan und Trifluormethyl substituiertes Phenylmethyl oder Phenylethyl stehen und

R ausserdem für einen gegebenenfalls durch Fluor, Chlor, Methoxy, Ethoxy, Methylthio, Ethylthio, Cyano oder Nitro substituierten Alkylrest mit 1 bis 6 Kohlenstoffatomen oder für $C_2$-$C_5$-Alkenyl steht.

**Claims**

1. Process for the preparation of N-substituted N-isocyanatocarbonyl-carbamates of the formula I

$$\begin{array}{l} R\text{-}N\text{-}COOR^1 \\ \quad | \\ \quad CO\text{-}NCO \end{array} \qquad (I)$$

in which

R and $R^1$ are identical or different and individually represent optionally substituted radicals from the aliphatic, cycloaliphatic, araliphatic or aromatic series,

characterised in that N-substituted carbamic acid esters of the formula II

$$R\text{-}NH\text{-}COOR^1 \qquad (II)$$

in which

R and $R^1$ have the abovementioned meaning,

are reacted with chlorocarbonyl isocyanate of the formula III

$$Cl\text{-}CO\text{-}NCO \qquad (III)$$

in a molar ratio of chlorocarbonyl isocyanate (III) to carbamic acid ester (II) of 1:1 to 10:1 at temperatures of 50 to 200°C in a diluent without the addition of an acid binder.

2. N-Substituted N-isocyanatocarbonyl carbamates of the formula Ia

$$\begin{array}{l} R\text{-}N\text{-}COOR^2 \\ \quad | \\ \quad CO\text{-}NCO \end{array} \qquad (Ia)$$

in which

R represents optionally substituted radicals from the aliphatic, cycloaliphatic, araliphatic or aromatic series, and

$R^2$ represents optionally substituted radicals from the cycloaliphatic, araliphatic or aromatic series.

3. N-Substituted N-isocyanatocarbonyl carbamates according to claim 2, wherein in the formula Ia

R and $R^2$ represent a cyclohexyl radical optionally substituted by methyl or ethyl, phenyl optionally substituted by fluorine, chlorine, methyl, ethyl, methoxy, ethoxy, n- and i-propoxy, nitro, cyano, trifluormethyl or dimethyldihydrofuranyl, naphthyl optionally substituted by fluorine, chlorine, methyl, ethyl, methoxy, ethoxy, n- and i-propoxy, nitro or trifluoromethyl, or phenylethyl or phenylmethyl optionally substituted in the phenyl part by methyl, ethyl, methoxy, ethoxy, fluorine, chlorine, nitro, cyano and trifluoromethyl and

R furthermore represents an alkyl radical which has 1 to 6 carbon atoms and is optionally substituted by fluorine, chlorine, methoxy, ethoxy, methylthio, ethylthio, cyano or nitro, or $C_2$-$C_5$-alkenyl.

## Revendications

1. Procédé de préparation de N-Isocyanatocarbonyl-carbamates N-substituées de formule I:

$$\begin{array}{l} R\text{-}N\text{-}COOR^1 \\ \quad | \\ \quad CO\text{-}NCO \end{array} \qquad (I)$$

dans laquelle

R et $R^1$ sont identiques ou différents et représentent chacun un radical éventuellement substitué choisi parmi la série aliphatique, cycloaliphatique, araliphatique ou aromatique,

caractérisé en ce qu'on fait réagir des esters d'acides carbamiques N-substitués de formule II:

$$R\text{-}NH\text{-}COOR^1 \qquad (II)$$

dans laquelle

R et $R^1$ ont les significations indiquées ci-dessus, avec du chlorocarbonylisocyanate de formule III:

$$Cl\text{-}CO\text{-}NCO \qquad (III)$$

dans un rapport molaire de 1:1 à 10:1 entre le chlorocarbonylisocyanate (III) et l'ester d'acide carbamique (II), à des températures de 50 à 200°C dans un diluant et sans addition d'un agent fixateur d'acide.

2. N-isocyanatocarbonyl-carbamates N-substitués de formula Ia:

$$\begin{array}{l} R\text{-}N\text{-}COOR^2 \\ \quad | \\ \quad CO\text{-}NCO \end{array} \qquad (Ia)$$

dans laquelle

R représente un radical éventuellement substitué choisi parmi la série aliphatique, cycloaliphatique, araliphatique ou aromatique, et

$R^2$ représente un radical éventuellement substitué choisi parmi la série cycloaliphatique, araliphatique ou aromatique.

3. N-isocyanatocarbonyl-carbamates N-substitués selon la revendication 2, caractérisés en ce que, dans la formula Ia:

R et $R^2$ représentent chacun un radical cyclohexyle éventuellement substitué par un radical méthyle ou par un radical éthyle, un radical phényle éventuellement substitué par le fluor, le chlore, le radical méthyle, le radical éthyle, le radical méthoxy, le radical éthoxy, le radical n-propoxy, le radical i-propoxy, le radical nitro, le radical cyano, le radical trifluorométhyle ou le radical diméthyldihydrofuranyle, le radical naphtyle éventuellement substitué par le fluor, le chlore, le radical méthyle, le radical éthyle, le radical méthoxy, le radical éthoxy, le radical n-propoxy, le radical i-propoxy, le radical nitro ou le radical trifluorométhyle, un radical phényléthyle ou un radical phénylméthyle éventuellement substitué dans la fraction phényle par le radical méthyle, le radical éthyle, le radical méthoxy, le radical éthoxy, le fluor, le chlore, le radical nitro, le radical cyano et le radical trifluorométhyle, et

R représente, en outre, un radical alkyle contenant 1 à 6 atomes de carbone et éventuellement substitué par le fluor, le chlore, le radical méthoxy, le radical éthoxy, le radical méthylthio, le radical éthylthio, le radical cyano ou le radical nitro, ou encore un radical alcényle en $C_2$-$C_5$.